# EUROPEAN PATENT APPLICATION

(11) **EP 4 595 964 A1**
(43) Date of publication of application: **06.08.2025**
(21) Application number: 24155342.9
(22) Date of filing: 01.02.2024
(51) Int. Cl.: A61K 31/445, A61P 25/00, A61P 25/28

(54) **TREATMENT OF GM1 GANGLIOSIDOSIS**

(71) Applicant: Azafaros B.V., 2333 CH Leiden (NL)
(72) Inventor: LANDSKRONER, Kyle, 4052 Basel (CH); WHEREAT, Jeremy, Brisbane, 4072 (AU); PIETROGRANDE, Giovanni, Brisbane, 4072 (AU); MORRISON, Sean, Brisbane, 4072 (AU); WOLVETANG, Ernst, Brisbane, 4072 (AU)
(74) Representative: Boult Wade Tennant LLP

(57) **Abstract**

The invention relates to a compound of formula (I), 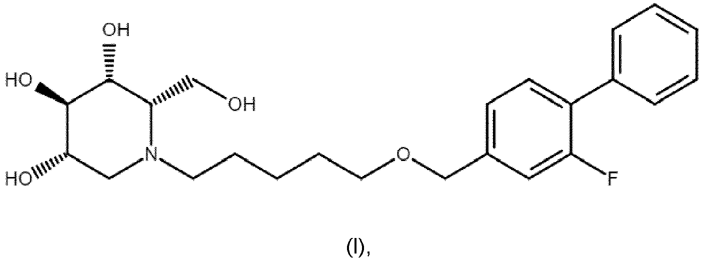 for use in treating GM1 gangliosidosis. Specifically, the invention relates to a compound of formula (I) for use in reducing the accumulation of GM1 ganglioside in the brain and/or spinal cord of a subject, increasing neuronal survival in a subject, and/or preventing neuronal degeneration in a subject.

## Description

### FIELD OF THE INVENTION

The invention relates to a compound of formula (I), 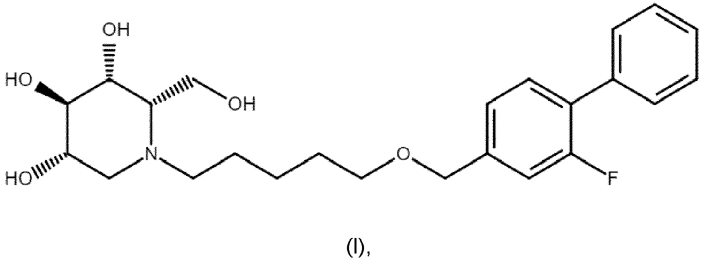 for use in treating GM1 gangliosidosis. Specifically, the invention relates to a compound of formula (I) for use in reducing the accumulation of GM1 ganglioside in the brain and/or spinal cord of a subject, increasing neuronal survival in a subject, and/or preventing neuronal degeneration in a subject.

### BACKGROUND OF THE INVENTION

GM1 gangliosidosis is an inherited lysosomal storage disorder that progressively destroys neurons in the brain and spinal cord. GM1 gangliosidosis is caused by genetic changes in the GLB1 gene and is inherited in an autosomal recessive manner. The GLB1 gene provides instructions for making β-galactosidase. This enzyme is found in lysosomes. β-galactosidase helps break down several molecules, including GM1 ganglioside. GM1 ganglioside is important for normal functioning of neurons in the brain.

The GLB1 gene variants produce versions of β-galactosidase that are not as effective at breaking down GM1 ganglioside as the normal version of the enzyme. Without enough functional β-galactosidase, GM1 ganglioside cannot be broken down when it is no longer needed. This substance builds up to toxic levels in many tissues and organs, particularly in the brain. Damage caused by the buildup of GM1 ganglioside leads to the destruction of neurons, causing many of the signs and symptoms of GM1 gangliosidosis.

The severity of symptoms and the time at which they first present can vary greatly in GM1 gangliosidosis. In general, an earlier manifestation of symptoms, as occurs in infantile or type I GM1 gangliosidosis, is often part of a more severe and rapidly progressive disease. Signs and symptoms children with GM1 gangliosidosis may experience include distinct facial features (coarse facial features), poor muscle tone (hypotonia), enlargement of the liver and spleen (hepatosplenomegaly), exaggerated startle reaction, developmental regression, skeletal abnormalities, dysmorphia, organomegaly, cardiomyopathy, interstitial lung disease, seizures, ataxia, dysarthria and visual impairment.

No treatment for GM1 gangliosidosis currently exists. Thus, a need exists for a therapy for GM1 gangliosidosis.

WO2015/147639 A1 describes a wide range of derivatives of deoxynojirimycin, including a compound of a formula (referred to as compound of formula (I) herein):

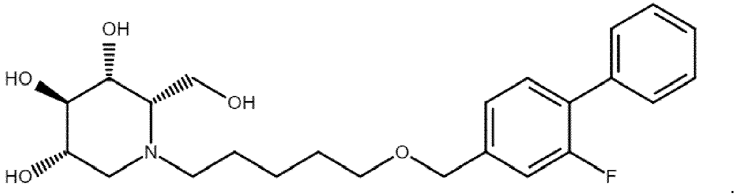

Compound of formula (I) is a potent dual inhibitor of glucosylceramide synthase and non-lysosomal glucosylceramidase (GBA2, UniProt code: Q9HCG7).

WO2022/069709 A1 describes two crystalline forms of compound of formula (I); "Form 3" and "Form 2". The X-ray diffraction pattern of the "Form 3" crystalline form is visualized in Figure 6A of WO2022/069709 A1. The X-ray diffraction pattern of the "Form 2" crystalline form is visualized in Figure 2 of WO2022/069709 A1.

### SUMMARY OF THE INVENTION

The inventors have tested the compound of formula (I) in the human induced pluripotent stem cell model of GM1 gangliosidosis and found that this compound is able to reduce GM1 ganglioside accumulation in GLB1 defective cerebral organoids in a dose-dependent fashion and concomitantly increased neuronal survival. The compound also shows no toxicity for the human brain cells.

In a first aspect, the invention provides a compound of formula (I), 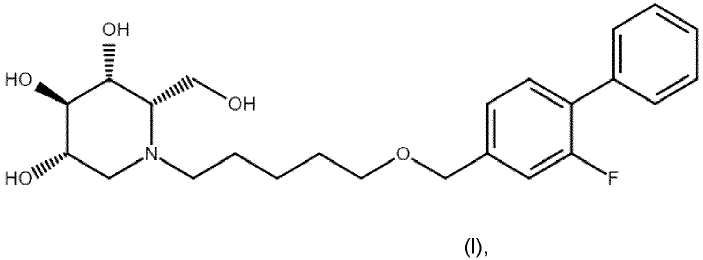 for use in treating GM1 gangliosidosis in a subject.

In a further aspect, the invention provides a compound of formula (I), 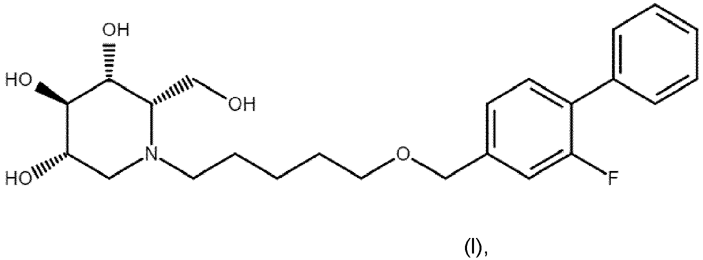 for use in reducing the accumulation of GM1 ganglioside in the brain and/or spinal cord of a subject, preferably wherein the subject suffers from GM1 gangliosidosis.

In a further aspect, the invention provides a compound of formula (I), 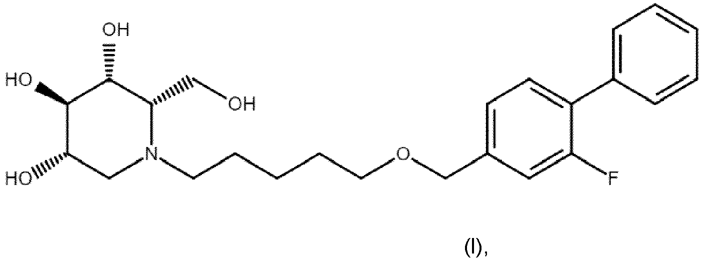 for use in increasing neuronal survival in a subject, preferably wherein the subject suffers from GM1 gangliosidosis.

In a further aspect, the invention provides a compound of formula (I), 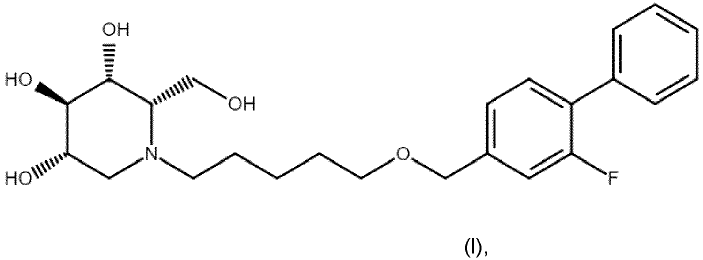 for use in preventing neuronal degeneration in a subject, preferably wherein the subject suffers from GM1 gangliosidosis.

The medical uses of the invention may be expressed in any suitable form. Thus, in a further aspect, the invention provides a method of treating GM1 gangliosidosis, the method comprising administering to the subject suffering from GM1 gangliosidosis a compound of formula (I): 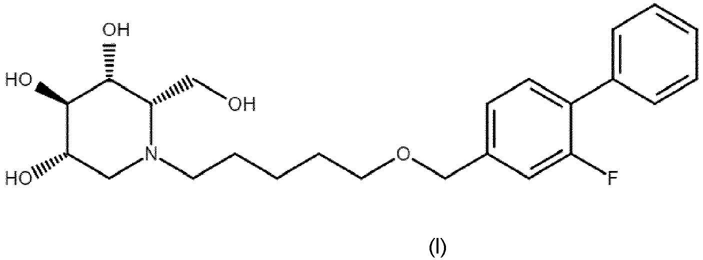 in an amount effective to treat GM1 gangliosidosis in the subject.

In a further aspect, the invention provides a use of a compound of formula (I) 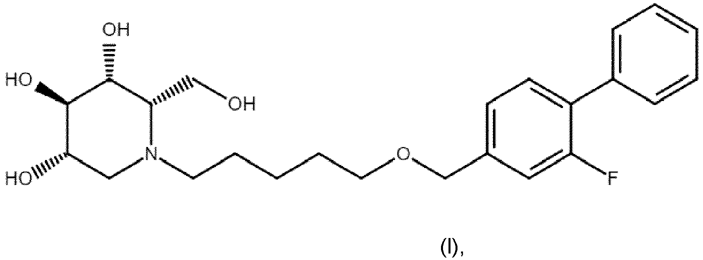 for producing a medicament for treating a subject suffering from GM1 gangliosidosis.

In a further aspect, the invention provides a use of a compound of formula (I) 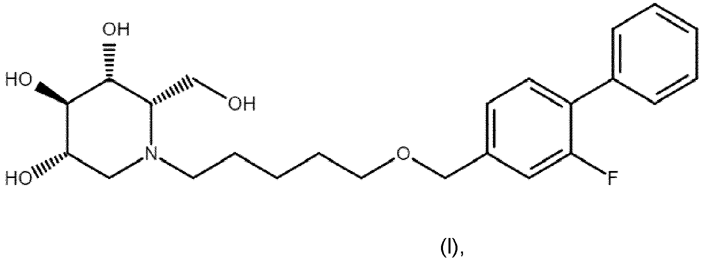 for producing a medicament for reducing the accumulation of GM1 ganglioside in the brain and/or spinal cord of a subject, preferably wherein the subject suffers from GM1 gangliosidosis.

In a further aspect, the invention provides a use of a compound of formula (I) 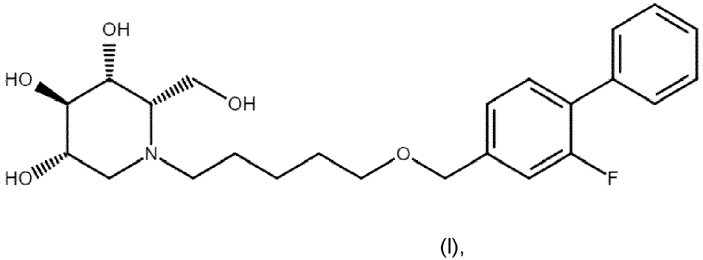 for producing a medicament for increasing neuronal survival in a subject, preferably wherein the subject suffers from GM1 gangliosidosis.

In a further aspect, the invention provides a use of a compound of formula (I) 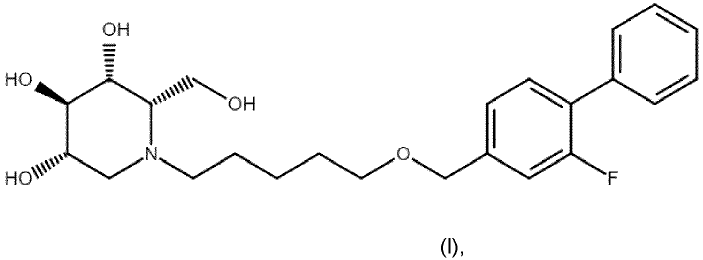 for producing a medicament for preventing neuronal degeneration in a subject, preferably wherein the subject suffers from GM1 gangliosidosis.

For any of the aspects described above, the compound of formula (I) is preferably in the crystalline form. Preferably, the crystalline form displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern. WO2022/069709 A1, which is incorporated herein by reference, describes suitable crystalline forms of compound of formula (I). Preferably, the compound of formula (I) is in "Form 3" described in WO2022/069709 A1.

Preferably, for any of the aspects described above, the compound is administered to the subject as a pharmaceutical composition. The pharmaceutical composition may comprise the compound together with at least one pharmaceutically acceptable carrier, diluent or excipient.

Other preferred embodiments of the compound for use according to the invention appear throughout the specification and in particular in the examples.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

### BRIEF DESCRIPTION OF THE DRAWINGS

These and other aspects of the invention will now be described with reference to the accompanying Figures, in which:

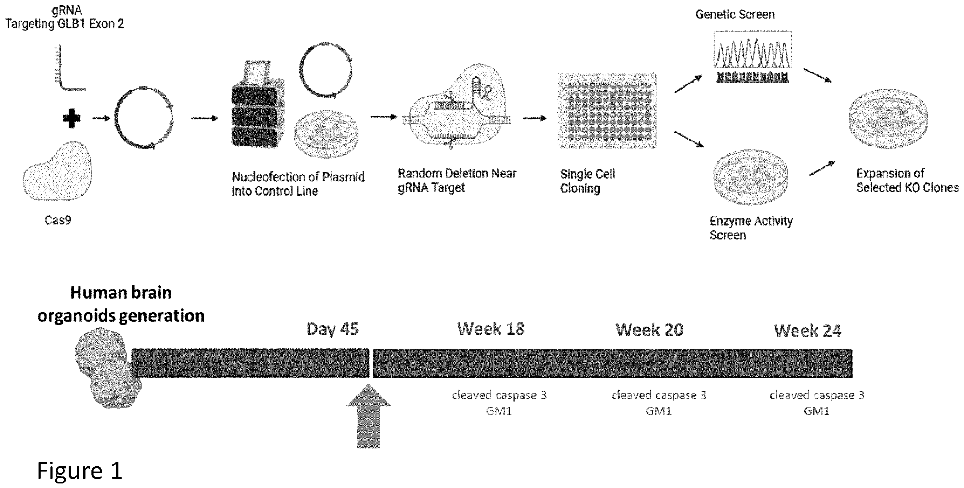

Figure 1 illustrates preparation of the GM1 gangliosidosis model in Human iPCs cells. Human iPCs cells G22 were transduced using a 4D-Nucleofector with a plasmid expressing CRISPR/Cas9 and a custom-designed guide RNA targeting exon 2 in the GLB1 locus. Single clones were generated and screened for the presence of homozygotic frameshift in *GLB1* coding sequence that would result in knockout, confirmed by absence of β-Galactosidase activity. The clone Ex2-3 was selected, expanded, and used to generate brain organoids containing microglia. After 45 days of maturation, WT and KO brain organoids were exposed to vehicle, 5nM or 500nM of compound of formula (I) (also termed "nizubaglustat" herein) for 14, 18, 20 and 24 weeks and processed for immunostaining. GM1 accumulation was measured using a cholera toxin subunit-B FITC conjugate.
Figure 2A illustrates the sequencing results of the GLB1 locus after homozygotic frameshift in GLB1 coding sequence (top panel). Due to the 1 bp deletion in clone 2.3, the elastin binding protein (EBP) will also be out of frame from amino acid 25 which would predict that this protein is either non-functional and/or unstable at the mRNA or protein level (bottom panel).

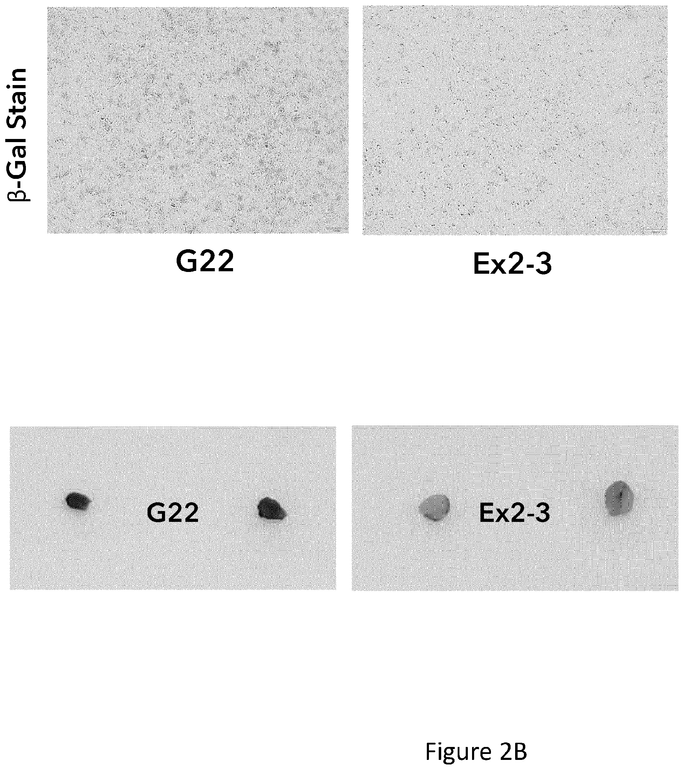

Figure 2B illustrates β-galactosidase (β-gal.) staining of the clonally derived Ex 2-3 GLB1 null line. In Figure 2B, top left, the blue β-gal. staining is present in the organoid tissue slices, confirming the activity of the enzyme. In contrast, the lack of substrate conversion correlates to a less blue colour, or β-gal enzyme present. The low levels of blue seen in the Ex 2-3 GLB1 null-line represent non-specific, background levels (Figure 2B, top right). Figure 2B bottom panel show the same effect, but for exemplary whole organoids (bottom, left, G22 control organoids and bottom, right, the EX 2-3 GLB1 null line).
Figure 2C illustrates the neuronal phenotype of the cells (Neun) as well as the presence of microglia (Iba1) and astrocytes (GFAP). DAPI was used to provide information on nucleated cells and helps to normalize quantitative analyses of specific markers.
Figure 3 (top panel) illustrates accumulation of GM1 ganglioside in *GLB1*-null cerebral organoids and control with or without nizubaglustat treatment. Figure 3 (bottom panel) shows the results of apoptosis tests with and without nizubaglustat.
Figure 4 (top panel) illustrates representative images take of organoids treated with vehicle or nizubaglustat at Week 24. Figure 4 (bottom panel) illustrates the results of the cellularity tests with and without nizubaglustat.

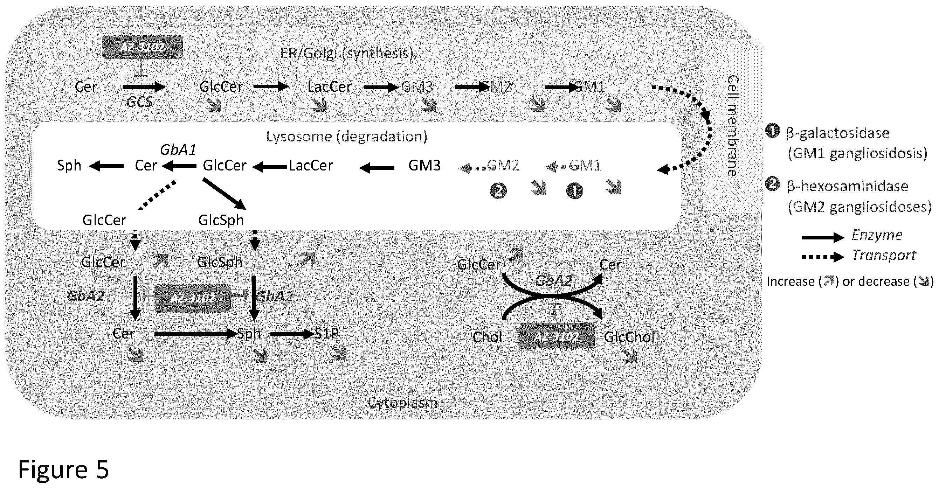

Figure 5 summarizes the mode of action of the crystalline form of compound of formula (I) (labelled as "AZ-3102").

### DETAILED DESCRIPTION OF THE INVENTION

Unless otherwise defined herein, scientific, and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition.

It should be understood that singular prepositions such as "a," "an," and "the," are often used for convenience, however, all instances of the singular are intended to encompass the plural unless otherwise indicated either explicitly or from context. The terms "comprising", "including" and "having" are intended to be inclusive and mean that there may be additional elements other than the listed elements. Further, it should be understood that all references, including journal articles, books, patents, technical documents, and the like, mentioned in this disclosure are hereby incorporated by reference in their entirety and for all purposes.

The term "about" as used herein for numerical data refers to a value within 10% of the underlying parameter (i.e., plus or minus 10%), and use of the term "about" at the beginning of a string of values modifies each of the values (i.e., "about 1, 2 and 3" refers to about 1, about 2 and about 3). For example, a temperature of "about 85 °C" can include temperatures between 75 °C and 95 °C.

The term "melting point" is well known in the art. The term "relatively high melting point" as used herein is intended to encompass crystalline forms that are stable enough to be formulated as pharmaceutical compositions.

The term "composition" as used herein is intended to encompass a product comprising the specified ingredients in the specified amounts, as well as any product which results, directly or indirectly, from combination of the specified ingredients in the specified amounts. Such term in relation to a pharmaceutical composition is intended to encompass a product comprising the compound of formula (I) and/or the crystalline form of compound of formula (I), and optionally additional ingredients that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition comprising a compound and/or a crystalline form of the present invention, and optionally a pharmaceutically acceptable carrier, diluent or excipient. By "pharmaceutically acceptable" it is meant the carrier, diluent or excipient (if present) must be compatible with the other ingredients of the formulation and not deleterious to the recipient thereof.

The terms "therapeutically effective amount" and "amount effective to" deliver the effect intend to encompass the amount of a compound, a crystalline form or a pharmaceutical composition that, when administered to a patient for treating a disease, is sufficient to effect such treatment for the disease. The terms "therapeutically effective amount" and "amount effective to" deliver the effect intend to encompass the amount of a compound, a crystalline form or a pharmaceutical composition that, when administered to a patient for delivering a therapeutic effect (e.g. increasing survival), is sufficient to achieve such therapeutic effect. The therapeutically effective amount will vary depending on the disease and its severity, and/or the age and weight of the patient. The term "subject" (or "patient") includes, but is not limited to, animals such as, for example, mammals. Preferably, the subject is a human.

The present invention provides a compound of formula (I), 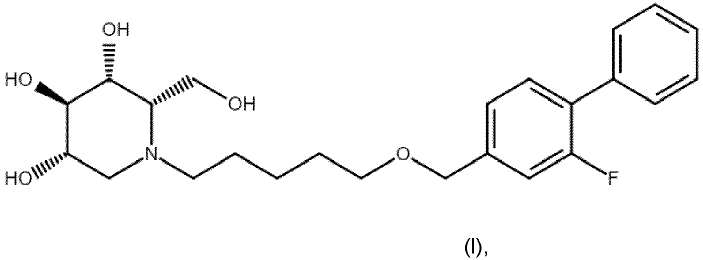 for use in treating GM1 gangliosidosis in a subject.

The invention provides a compound of formula (I), 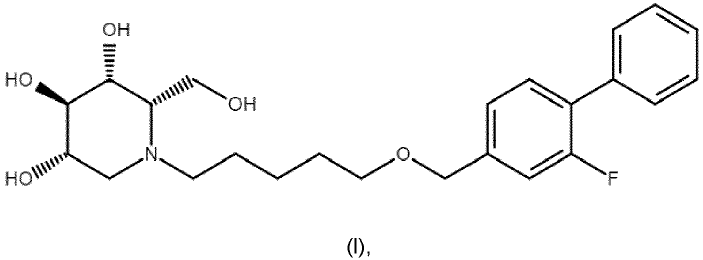 for use in eliminating, alleviating or ameliorating symptoms of GM1 gangliosidosis.

It will be appreciated that the treatment of GM1 gangliosidosis may result in the elimination, alleviation or amelioration of one or more symptoms of GM1 gangliosidosis and need not necessarily eliminate, alleviate or ameliorate every symptom of GM1 gangliosidosis that the patient experienced prior to treatment.

By 'elimination' of a symptom is meant that the subject essentially no longer experiences that symptom. By 'alleviation' of a symptom is meant that the subject experiences a significant improvement in the symptom. The alleviation is preferably from severe to mild. By 'amelioration' of a symptom is meant that the subject experiences a modest improvement in the symptom.

Thus, in particular embodiments, the treatment, use and the like provided herein may result in the elimination, alleviation or amelioration of one or more symptoms of GM1 gangliosidosis. For example, one or more symptoms may be alleviated and one or more different symptoms may be ameliorated.

As shown in the Examples, treatment with the compound of formula (I) reduced GM1 ganglioside accumulation in the cerebral organoids and increased neuronal survival and health in the human model of GM1 gangliosidosis. Thus, the compound of formula (I) may be used to eliminating, alleviating or ameliorating different symptoms of GM1 gangliosidosis.

The symptoms of GM1 gangliosidosis may include at least one of: distinct facial features (coarse facial features), poor muscle tone (hypotonia), enlargement of the liver and spleen (hepatosplenomegaly), exaggerated startle reaction, developmental regression, skeletal abnormalities, dysmorphia, organomegaly, cardiomyopathy, interstitial lung disease, seizures, visual impairment, ataxia, and/or dysarthria. The compound of formula (I) may be used to alleviate, eliminate and/or ameliorate at least one symptom of GM1 gangliosidosis in a subject suffering from GM1 gangliosidosis. Preferably, the compound of formula (I) alleviates, eliminates and/or ameliorates at least 2, at least 3, at least 4, or at least 5 symptoms of GM1 gangliosidosis.

When symptoms of GM1 gangliosidosis are present at birth or before 6 months of age, it is referred to as infantile or type I GM1 gangliosidosis. If the symptoms of GM1 gangliosidosis occur in late infancy or early childhood, it is referred to as type II GM1 gangliosidosis. When symptoms are present in adolescence or adulthood the disease is referred to as type III GM1 gangliosidosis.

The compound of formula (I) may be used to treat type I GM1 gangliosidosis, type II GM1 gangliosidosis, and/or type III GM1 gangliosidosis. The compound of formula (I) may be used in eliminating, alleviating or ameliorating the symptoms of type I GM1 gangliosidosis, type II GM1 gangliosidosis, and/or type III GM1 gangliosidosis.

The compound of formula (I) may be used to treat the symptoms of a disease showing phenotypic features of GM1 gangliosidosis. For example, the disease may be Morquio B disease. Morquio B disease is an attenuated phenotype within the spectrum of beta galactosidase (GLB1) deficiencies. It is characterised by dysostosis multiplex, ligament laxity, mildly coarse facies and heart valve defects due to keratan sulphate accumulation, predominantly in the cartilage. Morquio B patients have normal neurological development. The compound of formula (I) may be used to treat Morquio B disease.

Provided is also a method of eliminating, alleviating and/or ameliorating one or more of the above symptoms by administering a therapeutically effective amount of the compound of formula (I) to a subject in need thereof. The subject may suffer from GM1 gangliosidosis.

The medical uses of the invention may be expressed in any suitable form. Thus, in a further aspect, the invention provides a method of treating GM1 gangliosidosis, the method comprising administering to the subject suffering from GM1 gangliosidosis a compound of formula (I): 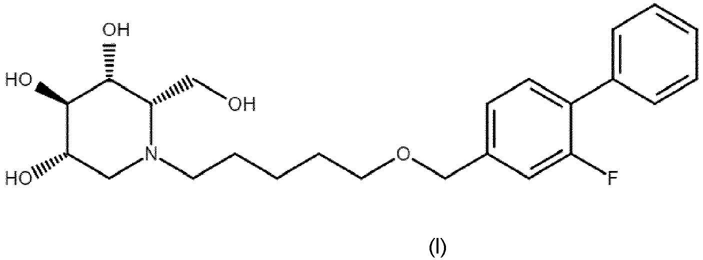 in an amount effective to treat GM1 gangliosidosis in the subject.

In a further aspect, the invention provides a use of a compound of formula (I) 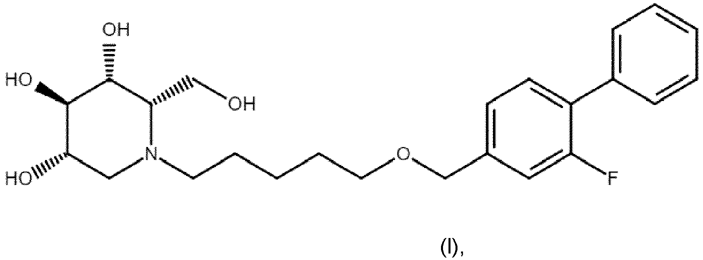 for producing a medicament for treating a subject suffering from GM1 gangliosidosis.

The invention also provides a compound of formula (I), 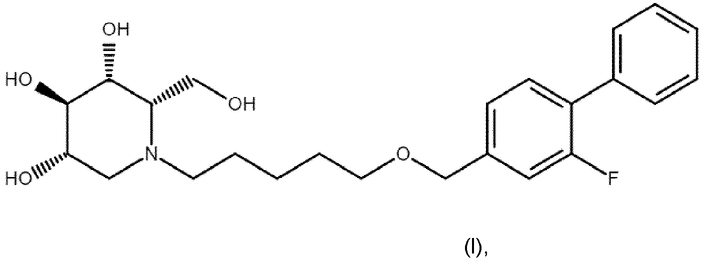 for use in reducing the accumulation of GM1 ganglioside in the brain and/or spinal cord of a subject, preferably wherein the subject suffers from GM1 gangliosidosis.

A method for reducing the accumulation of GM1 ganglioside in the brain and/or spinal cord of a subject is also provided, the method comprising administering to the subject a compound of formula (I) in an amount effective to reduce the accumulation of GM1 ganglioside in the brain and/or spinal cord of a subject suffering from GM1 gangliosidosis.

GM1 (monosialotetrahexosylganglioside) the "prototype" ganglioside, is a member of the ganglio series of gangliosides which contain one sialic acid residue. GM1 acts as the site of binding for both cholera toxin and E. coli heat-labile enterotoxin (Traveller's diarrhea). Methods suitable for determining the level of GM1 ganglioside are known to a person skilled in the art. Exemplary methods are described in the Examples. For example, the level of GM1 ganglioside may be measured by binding with a Cholera toxin subunit b fluorescein isothiocyanate (FITC) conjugate (C1655, Sigma) and using fluorescent microscopy or flow cytometry.

The expression "reducing the accumulation of GM1 ganglioside" encompasses the reduction of a GM1 ganglioside level in the brain and/or spinal cord of the subject after the treatment with the compound of formula (I) as compared to before the treatment. The reduction may take place after least 1, at least 2, at least 3, at least 4, at least 5, or at least 6 days of compound administration, or at least 1, at least 2, at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, or at least 30 weeks of regular (e.g. 2-3 times a week) compound administration.

The level of GM1 ganglioside accumulation in the brain and/or spinal cord of the subject may be reduced by at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, or at least 100% as compared to the level of GM1 ganglioside accumulation in the brain and/or spinal cord of the subject before the administration of the compound of formula (I). The level of GM1 ganglioside accumulation in the brain and/or spinal cord of the subject may be reduced at least 2-fold, at least 3-fold, at least 4-fold, or at least 5-fold as compared to the level of GM1 ganglioside accumulation in the brain and/or spinal cord of the subject before the administration of the compound of formula (I). The inventors of the present application have surprisingly discovered that compound of formula (I) (or crystalline form thereof) reduced the level of GM1 ganglioside at least 2-fold after 24 weeks of regular (2-3 times per week) administration of the compound.

The invention also provides a compound of formula (I), 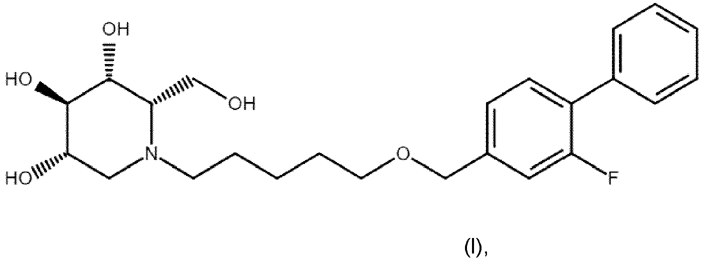 for use in increasing neuronal survival in a subject, preferably wherein the subject suffers from GM1 gangliosidosis.

A method for increasing neuronal survival in a subject is also provided, the method comprising administering to the subject a compound of formula (I) in an amount effective to increase neuronal survival in the subject suffering from GM1 gangliosidosis.

The compound of formula (I) may also be used to improve neuronal health.

The expression "neuronal survival" may mean that the life expectancy of a neuron after the treatment with the compound of formula (I) is improved by at least 10%, at least 20%, at least 30%, at least 40%, at least 50%, at least 60%, at least 70%, at least 80%, at least 90%, at least 100%, at least 200%, at least 300%, at least 400%, at least 500%, at least 600%, at least 700%, at least 800%, or at least 900% as compared to the life expectancy of the neuron without the treatment with the compound of formula (I). The neurons with increased survival may be from the brain and/or the spinal cord of the subject. By "increases survival" is meant an improvement in survival time compared to the situation in which the compound was not administered.

The invention provides a compound of formula (I), 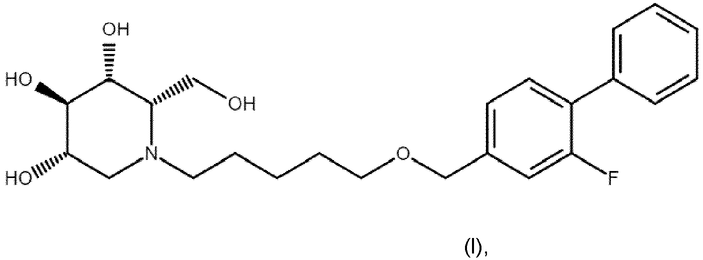 for use in preventing neuronal degeneration in a subject, preferably wherein the subject suffers from GM1 gangliosidosis.

A method for preventing neuronal degeneration in a subject is also provided, the method comprising administering to the subject a compound of formula (I) in an amount effective to prevent neuronal degeneration in the subject suffering from GM1 gangliosidosis.

The neuronal degeneration may be reduced after administration of the compound of formula (I) by at least 10%, at least 20%, at least 30%, at least 40%, at least 50%, at least 60%, at least 70%, at least 80%, at least 90%, at least 100% as compared to before the administration of the compound of formula (I).

The administration of the compound of formula (I) may stop neuronal degeneration for as long as the compound is administered to the subject in the therapeutically effective amount. The administration of the compound of formula (I) may slow neuronal degeneration for as long as the compound is administered to the subject in the therapeutically effective amount.

The invention provides a use of a compound of formula (I) 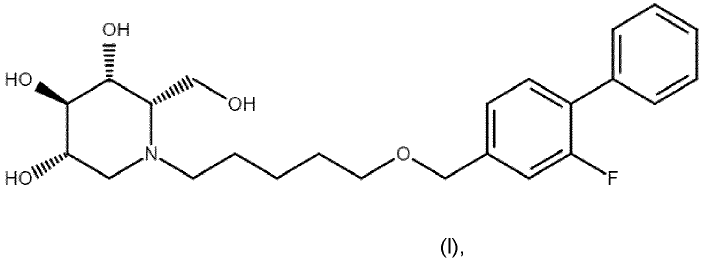 for producing a medicament for reducing the accumulation of GM1 ganglioside in the brain and/or spinal cord of a subject, preferably wherein the subject suffers from GM1 gangliosidosis.

The invention provides a use of a compound of formula (I) 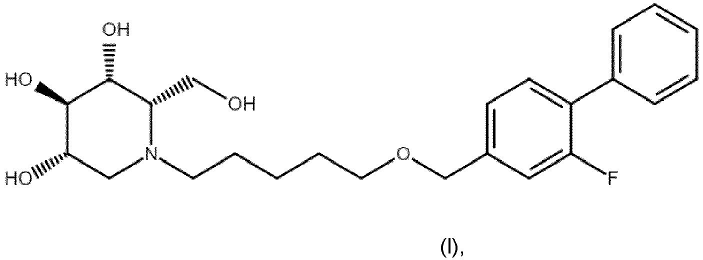 for producing a medicament for increasing neuronal survival in a subject, preferably wherein the subject suffers from GM1 gangliosidosis.

The invention provides a use of a compound of formula (I) 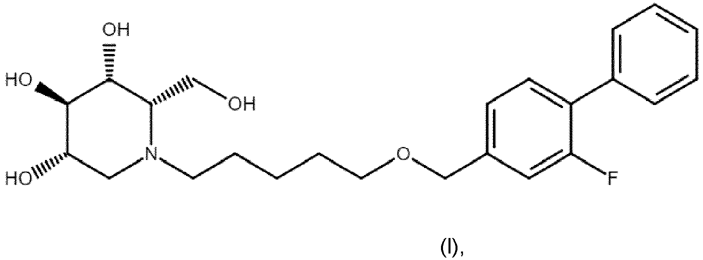 for producing a medicament for preventing neuronal degeneration in a subject, preferably wherein the subject suffers from GM1 gangliosidosis.

The compound of formula (I) may exhibit a substantial lack of toxicity in the subject. Preferably, the compound of formula (I) exhibits a substantial lack of brain toxicity in the subject. Preferably, the compound of formula (I) exhibits no brain toxicity in the subject. The inventors of the present application showed that the compound of formula (I) did not reduce cellularity nor induced apoptosis in the cells. This makes the compound of formula (I) (or the crystalline form thereof) particularly suitable for use in treating GM1 gangliosidosis.

For the avoidance of doubt, all embodiments specified herein are not limited to one particular wording to define the treatment and apply *mutatis mutandis* to the medical uses or methods of treatment aspects of the invention.

According to all aspects of the invention described herein, preferably, compound of formula (I) is in a crystalline form. The crystalline form of compound of formula (I) may be in any crystal state. The crystalline form of compound of formula (I) may be a crystalline salt or a crystalline free base (nonionized form). Preferably the crystalline form of compound of formula (I) is a crystalline free base. In addition, compound of formula (I) may form a co-crystal.

Preferably, the crystalline form displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern. WO2022/069709 A1, which is incorporated herein by reference, describes suitable crystalline forms of compound of formula (I). Preferably, the compound of formula (I) is in "Form 3" described in WO2022/069709 A1.

If the crystalline form of compound of formula (I) is a crystalline salt, the molecular structure of compound of formula (I) herein comprises a protonated nitrogen atom.

All aspects of the invention described herein are not limited to a single crystalline form of compound of formula (I). Solid materials may exist in more than one crystalline form. These alternative crystalline forms are termed polymorphs. Each polymorph has different orientations and/or conformations of molecules in the crystal lattice. Each crystal state, or "polymorph", exhibits a unique set of physicochemical properties due to differences in crystal structure.

Polymorphic forms may have different mechanical properties, such as fluidity and compressibility, which affect the technological properties of the compound. Stability and duration of storage of the compound may also depend on the polymorph.

Polymorphs can be distinguished from each other in different ways. Polymorphs clearly exhibit spectroscopic properties, and they can be determined using, for example, infrared spectroscopy, Raman spectroscopy, and ¹³C-NMR spectroscopy. In view of the fact that each crystalline form refracts X-rays in different ways, X-ray powder diffraction (XPD) can also be used to characterize polymorphs. In addition, thermal methods such as differential scanning calorimetry (DSC) can provide unique information on a particular polymorph.

As is well known in the field of powder X-ray diffraction, relative peak heights of powder X-ray diffraction spectra can be used to describe different crystalline forms. Accordingly, in a 'Form 3' aspect, the crystalline form of compound of formula (I) displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern. Preferably, the reflection at 17.8 ± 0.2° is one of the three strongest reflections in the X-ray powder diffraction pattern, or wherein the reflection at 17.8 ± 0.2° is one of the two strongest reflections in the X-ray powder diffraction pattern. More preferably, the reflection at 17.8 ± 0.2° is the strongest reflection in the X-ray powder diffraction pattern. More preferably still, in the 'Form 3' aspect, the crystalline form of compound of formula (I) displays a reflection, stated as a 2Θ value, at 17.8 ± 0.1°, in an X-ray powder diffraction pattern, wherein the reflection at 17.8 ± 0.1° is one of the four strongest reflections in the X-ray powder diffraction pattern. Preferably, the reflection at 17.8 ± 0.1° is one of the three strongest reflections in the X-ray powder diffraction pattern, or wherein the reflection at 17.8 ± 0.1° is one of the two strongest reflections in the X-ray powder diffraction pattern. More preferably, the reflection at 17.8 ± 0.1° is the strongest reflection in the X-ray powder diffraction pattern.

The term "strongest reflection" describes the highest peak of an X-ray powder diffraction pattern. The height of a peak of a powder X-ray diffraction pattern is determined based on the X-ray intensity (in counts or counts/sec units). Thus, the strongest reflection is a reflection that shows the highest X-ray intensity in the X-ray powder diffraction pattern.

Unless explicitly stated to the contrary, all X-ray powder diffraction patterns are determined using copper K-alpha radiation at about 25 °C.

Preferably, in the 'Form 3' aspect, the crystalline form of compound of formula (I) further displays one or more reflections, stated as a 2Θ value, at one or more of 4.1 ± 0.2°, 8.3 ± 0.2°, 12.4 ± 0.2°, 13.6 ± 0.2°, 14.5 ± 0.2°, 14.9 ± 0.2°, 15.2 ± 0.2°, 17.2 ± 0.2°, 19.3 ± 0.2°, 21.2 ± 0.2°, 22.4 ± 0.2°, 22.9 ± 0.2° and 23.3 ± 0.2°, in an X-ray powder diffraction pattern. More preferably still, in the 'Form 3' aspect, the crystalline form of compound of formula (I) further displays one or more reflections, stated as a 2Θ value, at one or more of 4.1 ± 0.1°, 8.3 ± 0.1°, 12.4 ± 0.1°, 13.6 ± 0.1°, 14.5 ± 0.1°, 14.9 ± 0.1°, 15.2 ± 0.1°, 17.2 ± 0.1°, 19.3 ± 0.1°, 21.2 ± 0.1°, 22.4 ± 0.1°, 22.9 ± 0.1° and 23.3 ± 0.1°, in an X-ray powder diffraction pattern.

The positions of peaks of powder X-ray diffraction spectra are relatively insensitive to experimental details. Thus, the crystalline compounds of the invention can be characterized by a powder X-ray diffraction pattern having certain peak positions. Accordingly, in the 'Form 3' aspect, the crystalline form of compound of formula (I) is preferably characterized by reflections, stated as a 2Θ value, at 17.2 ± 0.2°, 17.8 ± 0.2°, 21.2 ± 0.2° and 22.4 ± 0.2°, in an X-ray powder diffraction pattern. More preferably, in the 'Form 3' aspect, the crystalline form of compound of formula (I) is characterized by reflections, stated as a 2Θ value, at 4.1 ± 0.2°, 8.3 ± 0.2°, 12.4 ± 0.2°, 13.6 ± 0.2°, 14.5 ± 0.2°, 14.9 ± 0.1°, 15.2 ± 0.2°, 17.2 ± 0.2°, 17.8 ± 0.2°, 19.3 ± 0.2°, 21.2 ± 0.2°, 22.4 ± 0.2°, 22.9 ± 0.2° and 23.3 ± 0.2°, in an X-ray powder diffraction pattern. More preferably still, in the 'Form 3' aspect, the crystalline form of compound of formula (I) is characterized by reflections, stated as a 2Θ value, at 17.2 ± 0.1°, 17.8 ± 0.1°, 21.2 ± 0.1° and 22.4 ± 0.1°, in an X-ray powder diffraction pattern. Most preferably, in the 'Form 3' aspect, the crystalline form of compound of formula (I) is characterized by reflections, stated as a 20 value, at 4.1 ± 0.1°, 8.3 ± 0.1°, 12.4 ±0.1°, 13.6 ±0.1°, 14.5 ±0.1°, 14.9 ±0.1°, 15.2 ±0.1°, 17.2 ± 0.1°, 17.8 ± 0.1°, 19.3 ± 0.1°, 21.2 ± 0.1°, 22.4 ± 0.1°, 22.9 ± 0.1° and 23.3 ± 0.1°, in an X-ray powder diffraction pattern.

The crystalline forms of a compound can be characterized by a differential scanning calorimetry (DSC) thermogram. Thus, the crystalline form of compound of formula (I), in the 'Form 3' aspect, is preferably characterized by a DSC thermograph, which shows an onset of endothermic heat flow at about 87 °C and/or a melting point of about 92.4°C. Accordingly, preferably, in the 'Form 3' aspect, the crystalline form of compound of formula (I) has a melting point of 89 °C to 96 °C, as determined by DSC. Preferably, the crystalline form, in the 'Form 3' aspect, has a melting point of 90 °C to 95 °C, as determined by DSC. More preferably still the crystalline form of compound of formula (I), in the 'Form 3' aspect, has a melting point of 91 °C to 94 °C, as determined by DSC. Most preferably the crystalline form of compound of formula (I), in the 'Form 3' aspect, has a melting point of 92 °C to 93 °C, as determined by DSC.

A relatively high melting point (typically greater than about 80°C) of a therapeutic compound favours resistance to decomposition thereby facilitating the storage and increasing shelf life of the therapeutic compound, which is desirable for any pharmaceutical agent.

The crystalline forms of a compound can be characterized by their hygroscopicity. Hygroscopicity of a product expresses the increase or decrease in its water content as a function of relative humidity at a certain temperature. Substantially non-hygroscopic products exhibit no or only a slight change in their water content as a consequence of variations in relative humidity. In strongly hygroscopic products, water content may vary widely. Accordingly, preferably the crystalline form of compound of formula (I) is substantially non-hygroscopic. When preparing pharmaceutical compositions and formulations for use in such tablets, it is highly desirable to have a crystalline form of the therapeutic compound having low levels of hygroscopicity and/or low levels of deliquescence thereby allowing the compound to be compressed into a desired shape or size.

Substantially non-hygroscopic substances show a water absorption of less than about 2 % at a relative humidity of about 95% measured at a temperature of about 25 °C. Preferably, water absorption is less than about 1 % at a relative humidity of about 95% measured at a temperature of about 25 °C. The values of water absorption are obtained by measuring the mass gain of the tested crystalline form at a relative humidity of about 95% and a temperature of about 25 °C relative to the initial mass.

Accordingly, the crystalline form of compound of formula (I) preferably absorbs 0 % to 2 % water at a relative humidity of about 95% at a temperature of about 25 °C. More preferably still the crystalline form of compound of formula (I) absorbs 0 % to 1.5 % water at a relative humidity of about 95% at a temperature of about 25 °C. Most preferably the crystalline form of compound of formula (I) absorbs 0 % to 1 % water at a relative humidity of about 95% at a temperature of about 25 °C.

In a 'Form 2' aspect, the crystalline form of compound of formula (I) displays a reflection, stated as a 2Θ value, at 16.9 ± 0.2°, in an X-ray powder diffraction pattern, wherein the reflection at 16.9 ± 0.2° is one of the four strongest reflections in the X-ray powder diffraction pattern. Preferably, the reflection at 16.9 ± 0.2° is one of the three strongest reflections in the X-ray powder diffraction pattern, or wherein the reflection at 16.9 ± 0.2° is one of the two strongest reflections in the X-ray powder diffraction pattern. More preferably, the reflection at 16.9 ± 0.2° is the strongest reflection in the X-ray powder diffraction pattern. More preferably still, the crystalline form, in the 'Form 2' aspect, displays a reflection, stated as a 2Θ value, at 16.9 ± 0.1°, in an X-ray powder diffraction pattern, wherein the reflection at 16.9 ± 0.1° is one of the four strongest reflections in the X-ray powder diffraction pattern. Preferably, the reflection at 16.9 ± 0.1° is one of the three strongest reflections in the X-ray powder diffraction pattern, or wherein the reflection at 16.9 ± 0.1° is one of the two strongest reflections in the X-ray powder diffraction pattern. More preferably, the reflection at 16.9 ± 0.1° is the strongest reflection in the X-ray powder diffraction pattern.

Preferably, the crystalline form of compound of formula (I), in the 'Form 2' aspect, displays one or more reflections, stated as a 2Θ value, at one or more of 15.2 ± 0.2°, 16.1 ± 0.2°, 16.5 ± 0.2°, 18.9 ± 0.2°, 23.1 ± 0.2°, 25.5 ± 0.2°, 27.7 ± 0.2° and 28.5 ± 0.2°, in an X-ray powder diffraction pattern. More preferably still, in the 'Form 2' aspect, the crystalline form of compound of formula (I) displays one or more reflections, stated as a 2Θ value, at one or more of 15.2 ± 0.1°, 16.1 ± 0.1°, 16.5 ± 0.1°, 18.9 ± 0.1°, 23.1 ± 0.1°, 25.5 ± 0.1°, 27.7 ± 0.1° and 28.5 ± 0.1°.

This crystalline form of compound of formula (I), in the 'Form 2' aspect, can also be characterized by reflections, stated as a 2Θ value, at 16.1 ± 0.2°, 16.5 ± 0.2°, 16.9 ± 0.2°, 18.9 ± 0.2° and 23.1 ± 0.2°, in an X-ray powder diffraction pattern. Preferably, the crystalline form of compound of formula (I), in the 'Form 2' aspect, can also be characterized by reflections, stated as a 2Θ value, at 16.1 ± 0.1°, 16.5 ± 0.1°, 16.9 ± 0.1°, 18.9 ± 0.1° and 23.1 ± 0.1°, in an X-ray powder diffraction pattern.

The crystalline form of compound of formula (I), in the 'Form 2' aspect, can be characterized by a DSC thermograph which shows an onset of endothermic heat flow at about 58 °C and a melting point of about 70 °C. Accordingly, the crystalline form of compound of formula (I), in the 'Form 2' aspect, has a melting point of 67 °C to 74 °C. Preferably, the crystalline form of compound of formula (I), in the 'Form 2' aspect, has a melting point of 68 °C to 73 °C. More preferably still the crystalline form of compound of formula (I), in the 'Form 2' aspect, has a melting point of 69 °C to 72 °C. Most preferably the crystalline form of compound of formula (I), in the 'Form 2' aspect, has a melting point of 69 °C to 71 °C.

Preferably, the crystalline form of compound of formula (I), in the 'Form 2' aspect, is particularly soluble in water. Accordingly, preferably the crystalline form of compound of formula (I), in the 'Form 2' aspect, has water solubility of about 75 mg/mL to about 85 mg/mL, measured at about 25 °C. More preferably the crystalline form, in the 'Form 2' aspect, has water solubility of about 78 mg/mL to about 82 mg/mL, measured at about 25 °C. Most preferably the crystalline form of compound of formula (I), in the 'Form 2' aspect, has water solubility of about 80 mg/mL, measured at about 25 °C.

Methods for measuring solubility are known in the art; for example, shake-flask method, sonication, column elution method and ultraviolet or visible spectroscopy method. Unless explicitly stated to the contrary, water solubility is determined using the shake-flask method and/or sonication.

In some embodiments, the therapeutically effective amount (i.e. amount effective to deliver a desired effect) of the compound of formula (I) (or the crystalline form thereof) may be in the range of about 0.0001 milligrams total to about 50 milligrams total of the compound of formula (I) (or crystalline form thereof) per day (mg total/day). In specific embodiments, the therapeutically effective amount may be at least about 0.001 mg total/day, at least about 0.05 mg total/day, at least about 0.1 mg total/day, at least about 0.25 mg total/day, at least about 0.5 mg total/day, at least about 1 mg total/day, at least about 3 mg total/day, at least about 5 mg total/day, or at least about 8 mg total/day. In further embodiments, the therapeutically active amount may be in the range of about 0.01-20 mg total/day, 0.05-15 mg total/day or 0.1-12 mg total/day.

Thus, the compound of formula (I) (or the crystalline form thereof, preferably 'Form 3') may be administered to the subject at a dosage ranging from about 0.0001 mg total/day to about 50 mg total/day. Preferably, the compound of formula (I) (or the crystalline form thereof, preferably 'Form 3') is administered to the subject at a dosage ranging from about 0.1 mg total/day to about 15 mg total/day.

The dose of the compound of formula (I) (or the crystalline form thereof, preferably 'Form 3') throughout the treatment period of the subject may remain the same or it may be increased or decreased. The exact maintenance amount, according to all aspects of the invention, can be routinely determined by the skilled person based on the specific subject's physical condition, severity of GM1 gangliosidosis symptoms and tolerance of the compound being administered. Generally speaking, the exact maintenance amount will be a compromise between improvement of GM1 gangliosidosis symptoms against the physiological tolerance of the specific subject for that exact amount (e.g. the observance/potential for unwanted side effects). This is specific to each individual subject and it is routine for the skilled person in the art (e.g. a medical practitioner) to determine the exact maintenance amount for a specific subject based on the aforementioned factors.

In particular embodiments, the compound of formula (I) (or crystalline form thereof) may be administered as a single dose once per day. The compound of formula (I) (or crystalline form thereof) may be administered as two, three or more separate doses throughout the day. That is to say, the therapeutically effective amount per day is divided into two, three or more doses administered to the subject separately throughout the day. The multiple doses per day do not necessarily need to be the same amount.

The treatment (administration of the compound of formula (I), on a regular basis, such as daily) may be carried out for a suitable period of time, such as at least 2, 3, 4, 5, 6, 7, 8, 9 or 10 weeks, months or years. For example, the compound of formula (I) (or the crystalline form thereof) may be administered to the subject for at least 1 month, at least 3 months, at least 6 months, at least 9 months, at least 1 year, at least 2 years, at least 3 years. If it is well tolerated, the treatment may be carried out indefinitely. Alternatively, the compound of formula (I) (or the crystalline form thereof) may be administered to the subject one time.

Administration can be accomplished either by oral administration via tablets (such as dispersible tablets), pills, capsules, granules, powders, solutions, and the like, or parenteral administration, such as injections such as intra-articular, intravenous, and intramuscular injections, suppositories, ophthalmic solutions, eye ointments, or agents for external use, such as transdermal liquid preparations, ointments, transdermal patches, transmucosal liquid preparations, transmucosal patches, inhalers, and the like.

The subject treated or to be treated with the compound of formula (I) (or the crystalline form thereof) may exhibit one or more symptoms of GM1 gangliosidosis. Alternatively, the subject may be treated even if he or she does not exhibit any symptoms of GM1 gangliosidosis. The subject may be asymptomatic. The subject treated with the compound of formula (I) (or the crystalline form thereof) may have been diagnosed with GM1 gangliosidosis. The diagnosis of GM1 gangliosidosis may be performed based on genetic screening (e.g. to determine GLB1 gene mutations). Alternatively, the subject treated or to be treated with the compound of formula (I) (or the crystalline form thereof) might not have been diagnosed with GM1 gangliosidosis.

The one or more symptoms of GM1 gangliosidosis may include distinct facial features (coarse facial features), poor muscle tone (hypotonia), enlargement of the liver and spleen (hepatosplenomegaly), exaggerated startle reaction, developmental regression, skeletal abnormalities, dysmorphia, organomegaly, cardiomyopathy, interstitial lung disease, seizures, ataxia, dysarthria and visual impairment.

The subject treated or to be treated with the compound of formula (I) (or the crystalline form thereof) may have at least one mutation in a *GLB1* gene. The at least one mutation in the *GLB1* gene preferably results in an abnormal, deficient, or decreased activity of beta-galactosidase-1.

The subject treated or to be treated with the compound of formula (I) (or the crystalline form thereof) may exhibit abnormal or deficient activity of beta-galactosidase-1 as compared to a healthy subject (i.e. a subject not suffering from GM1 gangliosidosis). The subject treated or to be treated with the compound of formula (I) (or the crystalline form thereof) may exhibit decreased activity of beta-galactosidase-1 as compared to a healthy subject (i.e. a subject not suffering from GM1 gangliosidosis). The level of beta-galactosidase-1 may be measured in a sample from the subject using standard biochemical approaches.

According to all aspects of the invention, the subject is a mammal, preferably a human. In particular embodiments, the subject is between 28 days and 30 years of age. The subject may be an infant (i.e. between 28 days and 12 months of age). The subject may be a toddler (i.e. between 12 months and 24 months of age). The subject may be a child (i.e. between 24 months and 12 years of age). The subject may be an adolescent (i.e. between 12 years and 17 years of age). The subject may be a young adult (i.e. between 17 years and 30 years of age). The invention is, however, also applied to adults above 30 years of age in some embodiments.

The compound of formula (I) (or the crystalline form thereof) may be administered to the subject as a pharmaceutical composition. The pharmaceutical composition may have a pH value of between 2.0 and 8.0, between 3.0 and 7.0, between 4.0 and 7.5, between 4.0 and 6.0, or between 4.5 and 5.5. Preferably, the pharmaceutical composition has a pH value of between 4.5 and 5.5. The pharmaceutical composition may further comprise an acid and/or a base. For example, the pharmaceutical composition may comprise an acid to lower the pH to a desired pH value. The base may be used to increase the pH to a desired pH value. The skilled person would easily be able to determine suitable acids and bases to manipulate the pH value of the pharmaceutical composition. For example, the acid may be citric acid, acetic acid, or hydrochloric acid. For example, the base may be sodium hydroxide or potassium hydroxide.

The pharmaceutical compositions of the invention are typically prepared by pharmaceutically acceptable carrier and one or more optional ingredients. If necessary or desired, the resulting uniformly blended mixture can then be shaped or loaded into tablets (such as dispersible tablets), capsules, pills, canisters, cartridges, dispensers, and the like using conventional procedures and equipment.

When intended for oral administration in a solid dosage form (i.e., as capsules, tablets (such as dispersible tablets), pills and the like), the pharmaceutical compositions of the invention will typically comprise the compound of formula (I) (or the crystalline form thereof) as the active ingredient. The pharmaceutical composition of the invention may comprise the compound of formula (I) (or the crystalline form thereof) and no other ingredient. The pharmaceutical composition of the invention may be contained in a capsule. The pharmaceutical composition of the invention may be contained in the capsule without any other ingredient. The capsule may be a gelatine capsule or a hydroxypropyl methylcellulose (HPMC) capsule. Alternatively, the pharmaceutical composition of the invention may comprise the compound of formula (I) (or the crystalline form thereof) as the active ingredient and one or more pharmaceutically acceptable carriers. Suitable pharmaceutically acceptable carriers would be known by the person skilled in the art, for example, fats, water, physiological saline, alcohol (e.g., ethanol), glycerol, polyols, aqueous glucose solution, extending agent, disintegrating agent, binder, lubricant, wetting agent, stabilizer, emulsifier, dispersant, preservative, sweetener, colorant, seasoning agent or aromatizer, concentrating agent, diluent, buffer substance, solvent or solubilizing agent, chemical for achieving storage effect, salt for modifying osmotic pressure, coating agent or antioxidant, saccharides such as lactose or glucose; starch of corn, wheat or rice; fatty acids such as stearic acid; inorganic salts such as magnesium metasilicate aluminate or anhydrous calcium phosphate; synthetic polymers such as polyvinylpyrrolidone or polyalkylene glycol; alcohols such as stearyl alcohol or benzyl alcohol; synthetic cellulose derivatives such as methylcellulose, carboxymethylcellulose, ethylcellulose or hydroxypropylmethylcellulose, agents regulating the pH of the composition, such as bases and/or acids; and other conventionally used additives such as gelatin, talc, plant oil and gum arabic.

The oral dosage form may further be a capsule delayed release, in which the compound of formula (I) (or the crystalline form thereof) is enclosed within either a hard or soft soluble container made from a suitable form of gelatin or hydroxypropyl methylcellulose (HPMC), and which releases which the compound of formula (I) (or the crystalline form thereof) at a time other than promptly after administration, whereby enteric-coated articles are delayed release dosage forms. Capsule delayed release pellets, in which the compound of formula (I) (or the crystalline form thereof) is enclosed within either a hard or soft container or "shell" are also useful. In these cases, the compound of formula (I) (or the crystalline form thereof) itself is in the form of granules to which enteric coating has been applied, thus delaying release of the agent until its passing into the intestine. Capsule extended release and capsule film-coated extended release are also useful.

Additionally, the capsule may be covered in a designated film coating which releases the compound of formula (I) (or the crystalline form thereof) in such a manner to allow at least a reduction in dosing frequency as compared to that presented as a conventional dosage form. Examples include capsule gelatin coated (a solid dosage form in which the compound of formula (I) (or the crystalline form thereof) is enclosed within either a hard or soft soluble container made from a suitable form of gelatin or HPMC; through a banding process, the capsule is coated with additional layers of gelatin or HPMC so as to form a complete seal) and capsule liquid filled (a solid dosage form in which the compound of formula (I) (or the crystalline form thereof) is enclosed within a soluble, gelatin shell which is plasticized by the addition of a polyol, such as sorbitol or glycerin, and is therefore of a somewhat thicker consistency than that of a hard shell capsule).

In some embodiments, the compound of formula (I) (or the crystalline form thereof) may be dissolved or suspended in a liquid vehicle or formulated as a granule (a small particle or grain), a pellet (a small sterile solid mass consisting of a highly purified compound of formula (I) (or crystalline form thereof), with or without excipients, made by the formation of granules, or by compression and moulding), or a pellet coated extended release (a solid dosage form in which the compound of formula (I) (or the crystalline form thereof) itself is in the form of granules to which varying amounts of coating have been applied, and which releases the compound of formula (I) (or the crystalline form thereof) in such a manner to allow a reduction in dosing frequency as compared to that presented as a conventional dosage form).

Other forms include pills (a small, round solid dosage form containing the compound of formula (I) (or the crystalline form thereof) intended for oral administration), powder (an intimate mixture of dry, finely divided compound of formula (I) (or the crystalline form thereof) with one or more pharmaceutically acceptable additives that may be intended for internal or external use), elixir (a clear, pleasantly flavoured, sweetened hydroalcoholic liquid containing dissolved compound of formula (I) (or the crystalline form thereof); it is intended for oral use), chewing gum (a sweetened and flavoured insoluble plastic material of various shapes which when chewed, releases the compound of formula (I) (or the crystalline form thereof) into the oral cavity), syrup (an oral solution containing the compound of formula (I) (or the crystalline form thereof) and high concentrations of sucrose or other sugars; the term has also been used to include any other liquid dosage form prepared in a sweet and viscid vehicle, including oral suspensions), tablet (a solid dosage form containing the compound of formula (I) (or the crystalline form thereof) with or without suitable diluents), tablet chewable (a solid dosage form containing the compound of formula (I) (or the crystalline form thereof) with or without suitable diluents that is intended to be chewed, producing a pleasant tasting residue in the oral cavity that is easily swallowed and does not leave a bitter or unpleasant after-taste), tablet coated or tablet delayed release, dispersible tablet, tablet effervescent, tablet extended release, tablet film coated, or tablet film coated extended release where the tablet is formulated in such manner as to make the contained compound of formula (I) (or the crystalline form thereof) available over an extended period of time following ingestion. For example, the compound of formula (I) (or the crystalline form thereof) may be administered to a subject in the form of a dispersible tablet.

In other forms, a tablet for solution, tablet for suspension, tablet multilayer, tablet multilayer extended release may be provided, where the tablet is formulated in such manner as to allow at least a reduction in dosing frequency as compared to that presented as a conventional dosage form. A tablet orally disintegrating, tablet orally disintegrating delayed release, tablet soluble, tablet sugar coated, osmotic, and the like are also suitable.

As used herein, injectable and infusion dosage forms include, but are not limited to, a liposomal injectable, which either consists of or forms liposomes (a lipid bilayer vesicle usually composed of phospholipids which is used to encapsulate the compound of formula (I) (or the crystalline form thereof)); an injection, which includes a sterile preparation intended for parenteral use; an emulsion injection, which includes an emulsion consisting of a sterile, pyrogen-free preparation intended to be administered parenterally; or a lipid complex injection.

Other forms include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the compound of formula (I) (or the crystalline form thereof), either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

As used herein, a "solution injection" refers to a liquid preparation containing the compound of formula (I) (or the crystalline form thereof) dissolved in a suitable solvent or mixture of mutually miscible solvents that is suitable for injection. A "solution concentrate injection" refers to a sterile preparation for parenteral use which, upon the addition of suitable solvents, yields a solution conforming in all respects to the requirements for injections.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the compound of formula (I) (or the crystalline form thereof), either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

The parenteral carrier system includes one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the compound of formula (I) (or the crystalline form thereof) which is preferably isotonic with the blood of the recipient, but this is not essential.

As used herein, inhalation dosage forms include, but are not limited to, an aerosol (a product that is packaged under pressure and contains the compound of formula (I) (or the crystalline form thereof) which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the compound of formula (I) (or the crystalline form thereof), surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the compound of formula (I) (or the crystalline form thereof), in the form of a powder, that is released upon activation of an appropriate valve system. An aerosol spray is an aerosol product which utilizes a compressed gas as the propellant to provide the force necessary to expel the product as a wet spray and being applicable to solutions of the compound of formula (I) (or the crystalline form thereof) in aqueous solvent(s).

As discussed briefly above, the pharmaceutical composition comprising the crystalline forms of compound (I) can also be administered transdermally or transmucosally using known delivery systems and excipients. For example, the pharmaceutical composition can be admixed with permeation enhancers such as propylene glycol, polyethylene glycol monolaurate, azacycloalkan-2-ones and the like, and incorporated into a patch or similar delivery system. Additional excipients including gelling agents, emulsifiers, and buffers, may also be used. As used herein, transdermal dosage form includes, but is not limited to, a patch (a drug delivery system that often contains an adhesive backing that is usually applied to an external site on the body, whereby the ingredients (including the compound of formula (I) (or the crystalline form thereof)) either passively diffuse from, or are actively transported from, some portion of the patch, and whereby depending upon the patch, the ingredients (including the compound of formula (I) (or the crystalline form thereof)) are either delivered to the outer surface of the body or into the body. Various types of transdermal patches such as matrix, reservoir and others are known in the art.

As used herein, the topical dosage form includes various dosage forms known in the art such as lotions (an emulsion, liquid dosage form, whereby this dosage form is generally for external application to the skin), lotion augmented (a lotion dosage form that enhances delivery of the compound of formula (I) (or the crystalline form thereof), whereby augmentation does not refer to the strength of the compound of formula (I) (or the crystalline form thereof) in the dosage form), gels (a semisolid dosage form that contains a gelling agent to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances compound delivery, whereby augmentation does not refer to the strength of the compound of formula (I) (or the crystalline form thereof) in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances compound delivery, whereby augmentation does not refer to the strength of the compound of formula (I) (or the crystalline form thereof) in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the compound of formula (I) (or the crystalline form thereof) and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

Preferably the pharmaceutical composition comprises one or more further pharmaceutically active agents (e.g. anti-inflammatory agents, such as Aspirin). This combination therapy involves using a crystalline form of compound (I) combined with one or more of the further pharmaceutically active agents, either formulated together (for example, packaged together in a single formulation) or formulated separately (for example, packaged as separate unit dosage forms).

The methods and uses described herein may be *in vitro* methods (or uses) or *in vivo* methods (or uses).

The above methods and uses may be combined with a method of diagnosis of GM1 gangliosidosis, in which GM1 gangliosidosis is diagnosed first in a subject, followed by administration of the compound of formula (I) (or the crystalline form thereof) to deliver a desired therapeutic effect to the subject suffering from GM1 gangliosidosis. The method of diagnosis may comprise genetic screening of GLB1 gene to determine the presence of at least one mutation. The method of diagnosis may comprise determining the levels of beta-galactosidase-1 in a subject.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

### EXAMPLES

The crystalline form of compound (I) ("Form 3") has been obtained as described in WO2022/069709 A1. This crystalline form is referred to as "nizubaglustat" in the Examples.

### Example 1

### 1. Methods

As visualized in Figure 1, human iPCs cells G22 (Shaker et al. 2022) were transduced using a 4D-Nucleofector (Lonza) with a plasmid expressing CRISPR/Cas9 and a custom-designed guide RNA targeting exon 2 in the GLB1 locus (#62988, Addgene) as described in (Shaker et al. 2021). Single clones were generated and screened for the presence of homozygotic frameshift in *GLB1* coding sequence that would result in knockout, confirmed by absence of β-Galactosidase activity. The clone Ex2-3 was selected, expanded, and used to generate brain organoids containing microglia as described in (Aguado et al., 2023). After 45 days of maturation, WT and KO brain organoids were exposed to vehicle, 5nM or 500nM of nizubaglustat for 14, 18, 20 and 24 weeks and processed for immunostaining. GM1 accumulation was measured using a cholera toxin subunit-B FITC conjugate (C1655, Sigma)

### 2. Results & conclusions

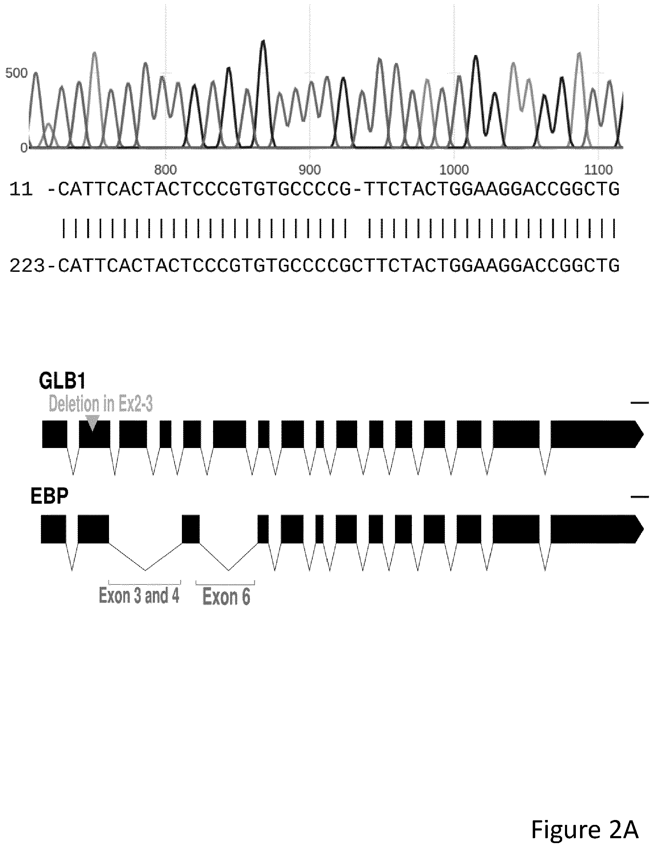

To evaluate the efficacy of nizubaglustat, a novel and potent dual NLGase (GBA2) and GCS inhibitor currently in clinical development, and to create a tool for mechanistic studies, a human brain organoid model of GM1 gangliosidosis has been developed. Using CRISPR-Cas9, a bi-allelic 1 base pair (bp) frameshift deletion in Exon 2 of the *GLB1* gene in healthy hESC was created to result in a non-functional β-galactosidase enzyme (Figure 2A). Due to the 1 bp deletion in clone 2.3, the elastin binding protein (EBP) will also be out of frame from amino acid 25 which would predict that this protein is either non-functional and/or unstable at the mRNA or protein level.

As visualized in Figure 2B, β-galactosidase (β-gal.) staining of the clonally derived Ex 2-3 GLB1 null line confirmed the absence of β-gal. enzyme activity. The β-gal. enzyme converts an exogenous substrate (chromogen) to a blue color when the enzyme is present. In Figure 2B, top left, the blue β-gal. staining is present in the organoid tissue slices, confirming the activity of the enzyme. In contrast, the lack of substrate conversion correlates to a less blue color, or β-gal enzyme present. The low levels of blue seen in the Ex 2-3 GLB1 null-line represent non-specific, background levels (Figure 2B, top right). Figure 2B bottom figures show the same effect, but for exemplary whole organoids (bottom, left, G22 control organoids and bottom, right, the EX 2-3 *GLB1* null line).

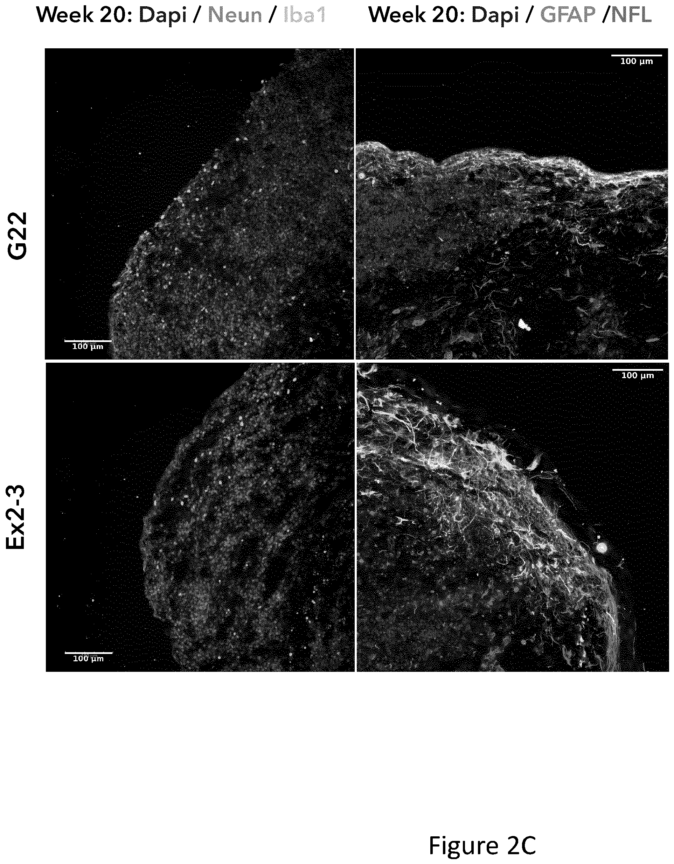

Figure 2C illustrates the neuronal phenotype of the cells (Neun) as well as the presence of microglia (Iba1) and astrocytes (GFAP). Neun is associated with neuronal differentiation and a common marker used to indicate a neuronal phenotype that is present throughout the whole cell life. Iba1 (Ionized calcium-binding adaptor molecule 1) is a pan microglial marker and it's expression increases with microglial activation. Glial fibrillary acidic protein (GFAP), is expressed by numerous cell types, including astrocytes. DAPI was used to provide information on nucleated cells and helps to normalize quantitative analyses of specific markers.

Next, >400 cerebral organoids from the Ex 2-3 line and Gen22, the isogenic parental control line, were generated, and these were cultured in the presence of 5 nM, 500 nM nizubaglustat or vehicle-control from 6 to 24 weeks. Analysis of organoid size revealed the presence of neurons (NFL and Neun +ve), astrocytes (GFAP +ve) and microglia (!BA1 +ve) at later timepoints in treated and untreated GLB1-null and control organoids.

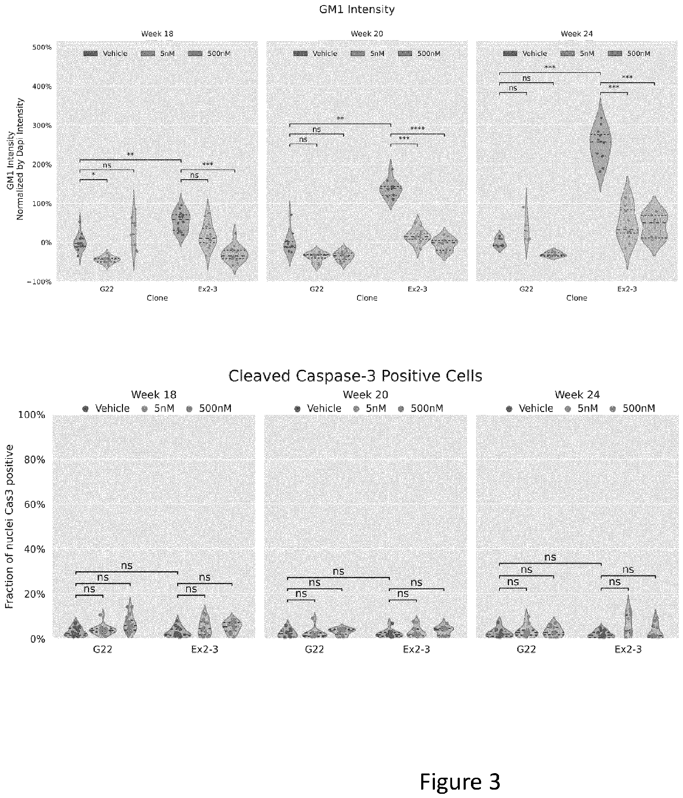

As illustrated in Figure 3 (top panel), the *GLB1*-null cerebral organoids exhibited progressive accumulation of GM1 as compared to the isogenic control organoids that became significant by week 18. Importantly, treatment with 5 nM, 500 nM nizubaglustat significantly reduced GM1 accumulation in *GLB1*-null Ex 2-3 cerebral organoids at weeks 18, 20 and 24 in a dose-dependent fashion and concomitantly increased neuronal survival and health in this human model of GM1 gangliosidosis.

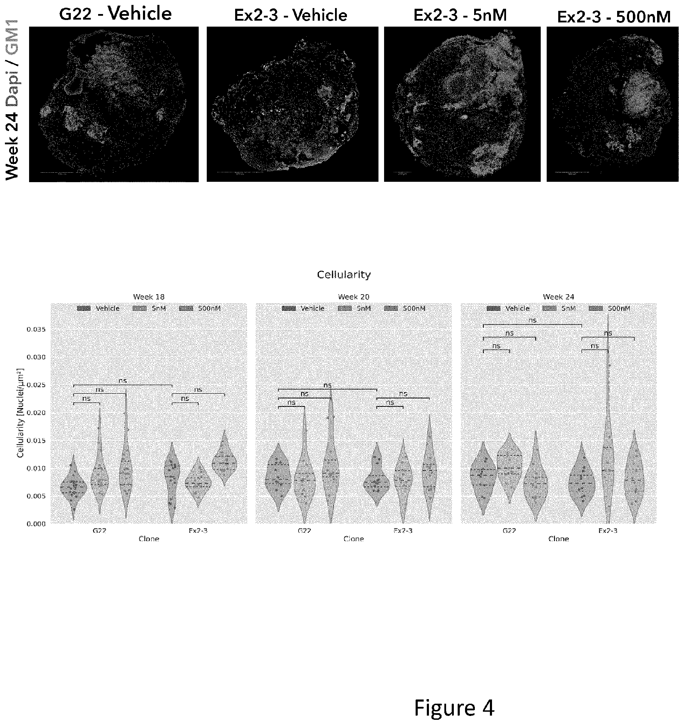

As illustrated in Figure 3 (bottom panel) and Figure 4 (bottom panel), exposure of control or *GLB1*-null cerebral organoids to nizubaglustat did not reduce cellularity nor induce apoptosis (cleaved caspase3 +ve cells) above baseline levels at any timepoint, suggesting the compound has no evident toxicity for the human brain cell types present in this model.

Figure 4 (top panel) illustrates representative images taken of organoids treated with vehicle or nizubaglustat at Week 24. The green stain represents GM1 levels, which are quite low in the G22 cell line, but significantly increase with the GLB1-null (Ex2-3) cell line. Treating cells with nizubaglustat significantly reduces the level of GLB1 in the EX 2-3 cell line.

### Conclusions

- Using CRISPR-Cas9, a bi-allelic 1 bp frameshift deletion in Exon 2 of the GLB1 gene was created in healthy hESC
- β-galactosidase staining confirmed the absence of β-gal. enzyme activity and the position of the mutation likely disrupts the EBP (elastin binding protein) function
- *GLB1*-null cerebral organoids exhibited progressive accumulation of GM1 (as measured by CTXb-streptavidin FITC) that became significant by WK 18
- Treatment with 5 nM, 500 nM nizubaglustat, did not reduce cellularity nor induce apoptosis (cleaved caspase3 positive cells) above baseline levels at any timepoint
- Treatment with 5 nM or 500 nM nizubaglustat significantly reduced GM1 accumulation in *GLB1*-null Ex 2-3 cerebral organoids at WK18, 20 and 24 in a dose-dependent fashion
- Nizubaglustat, a novel, dual inhibitor of the non-lysosomal neutral glucosylceramidase (NLGase, GBA2) and ceramide glucosyltransferase (GCS), has no evident toxicity for the human brain cell types present in this model, was effective in reducing GM1 and has great potential to become an effective therapy for GM1 gangliosidosis

### 3. References

1) Shaker MR, Kahtan A, Prasad R, Lee JH, Pietrogrande G, Leeson HC, et al. Neural Epidermal Growth Factor-Like Like Protein 2 Is Expressed in Human Oligodendroglial Cell Types. Front Cell Dev Biol. 2022;10:803061.
2) Shaker MR, Pietrogrande G, Martin S, Lee JH, Sun W, and Wolvetang EJ. Rapid and Efficient Generation of Myelinating Human Oligodendrocytes in Organoids. Front Cell Neurosci. 2021;15:631548.
3) Aguado J, Amarilla AA, Taherian Fard A, Albornoz EA, Tyshkovskiy A, Schwabenland M, Pietrogrande G, et al. Senolytic therapy alleviates physiological human brain aging and COVID-19 neuropathology. Nat Aging. 2023;3(12):1561-75.

## Claims

1. A compound of formula (I), 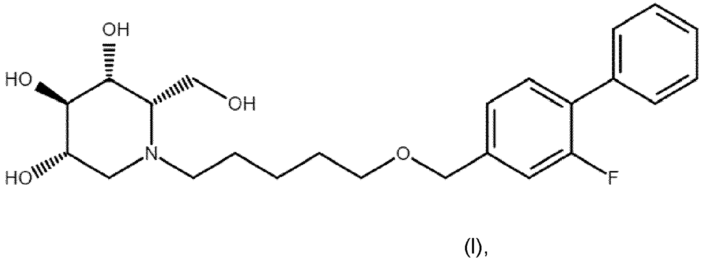 for use in treating GM1 gangliosidosis in a subject.

2. A compound of formula (I), 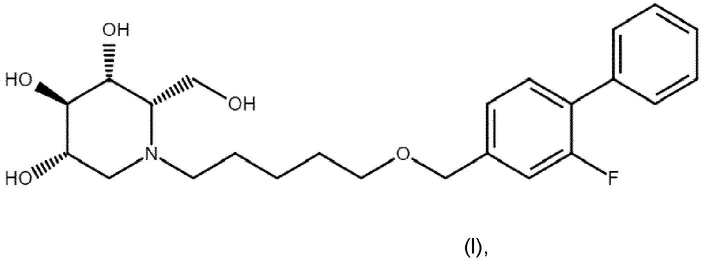 for use in reducing the accumulation of GM1 ganglioside in the brain and/or spinal cord of a subject, preferably wherein the subject suffers from GM1 gangliosidosis.

3. A compound of formula (I), 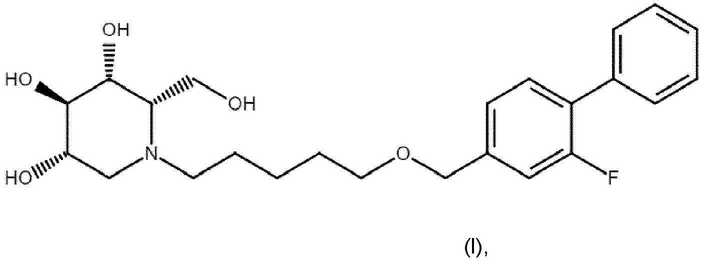 for use in increasing neuronal survival in a subject, preferably wherein the subject suffers from GM1 gangliosidosis.

4. A compound of formula (I), 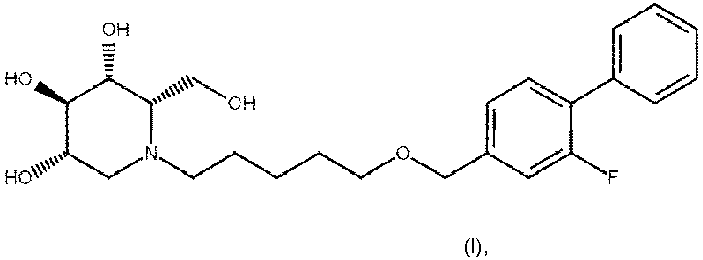 for use in preventing neuronal degeneration in a subject, preferably wherein the subject suffers from GM1 gangliosidosis.

5. The compound for use of any one of claims 1-4, wherein the compound is in a crystalline form.

6. The compound for use of claim 5, wherein the crystalline form is a crystalline free base.

7. The compound for use of claim 5 or claim 6, wherein the crystalline form displays a reflection, stated as a 2Θ value, at 17.8 ± 0.2°, in an X-ray powder diffraction pattern, wherein the reflection at 17.8 ± 0.2° is one of the four strongest reflections in the X-ray powder diffraction pattern.

8. The compound for use of claim 7, further displaying one or more reflections, stated as a 2Θ value, at one or more of 4.1 ± 0.2°, 8.3 ± 0.2°, 12.4 ± 0.2°, 13.6 ± 0.2°, 14.5 ± 0.2°, 14.9 ± 0.2°, 15.2 ± 0.2°, 17.2 ± 0.2°, 19.3 ± 0.2°, 21.2 ± 0.2°, 22.4 ± 0.2°, 22.9 ± 0.2° and 23.3 ± 0.2°, in an X-ray powder diffraction pattern.

9. The compound for use of any one of claims 1-8, wherein the compound is administered to the subject at a dosage ranging from about 0.1 mg total/day to about 15 mg total/day.

10. The compound for use of any one of claims 1-9, wherein the compound is administered to the subject daily.

11. The compound for use of any one of claims 1-10, wherein the subject:
(a) exhibits one or more symptoms of GM1 gangliosidosis;
(b) does not exhibit any symptoms of GM1 gangliosidosis;
(c) has been diagnosed with GM1 gangliosidosis; or
(d) has not been diagnosed with GM1 gangliosidosis.

12. The compound for use of any one of claims 1-11, wherein the subject is human, preferably the subject is between 28 days and 30 years of age.

13. The compound for use of any one of claims 1-12, wherein the compound is administered to the subject in a pharmaceutical composition which has a pH of between 4.0 and 7.5.

14. The compound for use of any one of claims 1-13, wherein the compound exhibits a substantial lack of brain toxicity in the subject.
